# EUROPÄISCHE PATENTSCHRIFT

(11) **EP 0 453 920 B1**
(45) Veröffentlichungstag und Bekanntmachung des Hinweises auf die Patenterteilung: **08.12.1993**
(21) Anmeldenummer: 91106001.0
(22) Anmeldetag: 16.04.1991
(51) Int. Cl.: C07C 29/80, C07C 33/14

(54) **Verfahren zur Reinigung von alpha-Bisabolol**
Method for the purification of alpha-bisabolol
Procédé pour l'épuration d'alpha-bisabolol

(30) Priorität: 24.04.1990 DE 4012945
(43) Veröffentlichungstag der Anmeldung: 30.10.1991
(73) Patentinhaber: BASF Aktiengesellschaft, 67063 Ludwigshafen (DE)
(72) Erfinder: Lauterbach, Gerald, Dr., W-6140 Bensheim (DE); Hertel, Otto, Dr., W-6700 Ludwigshafen (DE); Euler, Klaus, Dr., W-6700 Ludwigshafen (DE)

(56) Entgegenhaltungen:
- EP-A- 0 253 922
- BE-A- 755 564
- DE-B- 2 418 166
- Perfume & Essential Oil Record, November 1958, GB, Seiten 711-714;O.R. Gottlieb: "Essential Oil of the Wood of Vanillosmopsis erythropappa Schultz-Bip."
- World Patent Index Latest database, Derwent Publ London, GB. Accession Nr. 89-330177, Wk. 45 1989 & JP-A-01-247495 (03.10.89) (Eikodo)

## Beschreibung

Die vorliegende Erfindung betrifft ein neues Verfahren zur Reinigung von α-Bisabolol durch Destillation eines α-Bisabolol enthaltenden Pflanzenextraktes unter vermindertem Druck.

Das α-Bisabolol ist ein Sesquiterpenalkohol der folgenden Formel 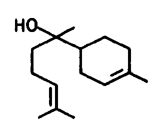
welcher in vier stereoisomeren Formen vorkommt.

Aus der DE-A 2 317 583 und der EP-A 253 922 sind Verfahren zur Gewinnung und Reinigung von α-Bisabolol aus Pflanzenextrakten bekannt, in welchen man die Extrakte durch Waschen mit alkalischen Lösungen und Behandlung mit Aktivkohle oder durch Filtrieren über ein Ionenaustauscher-Harz vorreinigt und anschließend einer Destillation unterwirft. In beiden Druckschriften wird darauf hingewiesen, daß sich α-Bisabolol durch Destillation allein nicht reinigen lasse.

Da diese Verfahren technisch sehr aufwendig sind, lag der Erfindung ein einfacheres und wirtschaftlicheres Verfahren zur Reinigung von α-Bisabolol als Aufgabe zugrunde.

Demgemäß wurde ein Verfahren zur Reinigung von α-Bisabolol durch Destillation eines α-Bisabolol enthaltenden Pflanzenextraktes unter vermindertem Druck gefunden, welches dadurch gekennzeichnet ist, daß man das gereinigte α-Bisabolol in der unteren Hälfte einer Destillationskolonne als Seitenprodukt abzieht und den Extrakt oberhalb des Seitenabzugs zuführt.

Die α-Bisabolol enthaltenden Extrakte können nach an sich bekannten Methoden aus verschiedenen Pflanzen wie Matricaria chamomilla (M.A. Schwartz, G.C. Swanson; J. Org. Chem. 44 (1979) 953) und bevorzugt aus Vanillosmopsis erythropappa (B.R. Gottlieb, M.T. Magalhaes; Perf. Essent. Oil. Rec. (1958) 711) isoliert werden. Das aus Vanillosmopsis erythropappa isolierte Extrakt ist unter der Bezeichnung Candeia-Öl bekannt.

In der Regel enthalten die Rohextrakte das α-Bisabolol in Mengen von 70 bis 90 Gew.-%. Als weitere Inhaltsstoffe enthalten sie unangenehm riechende und farblich störende Substanzen wie Isovaleriansäure, Bisabolen und Eremanthin.

Die für das erfindungsgemäße Verfahren geeigneten Destillationskolonnen weisen in der Regel 6 bis 50, insbesondere 10 bis 30 theoretische Böden auf. Auf die Bauart der Kolonne kommt es nicht an, d.h. man kann Glockenboden-, Siebboden-, Ventilboden- und Gitterbodenkolonnen oder vorzugsweise Füllkörperkolonnen verwenden.

Bei Füllkörperkolonnen kann man alle üblichen Kolonnenpackungen wie Raschigringe, Pallringe oder Metallpackungen einsetzen.

Man führt den Rohextrakt vorzugsweise im Bereich der Höhe von zwei Dritteln der Kolonne zu. Die Entnahmestelle für das α-Bisabolol liegt im Bereich der Höhe des ersten Drittels der Kolonne und mindestens 2 theoretische Böden unterhalb der Zugabestelle des Rohextraktes. Üblicherweise werden Zulauf und Seitenabzug so angeordnet, daß zwischen ihnen und zwischen Kolonnensumpf und Seitenabzug je 2 bis 20 theoretische Böden liegen.

Das Rücklaufverhältnis wird der gewünschten Produktqualität angepaßt, indem man es in der Regel im Bereich von 2:1 bis 20:1, vorzugsweise im Bereich von 3:1 bis 12:1 wählt.

Man fraktioniert die Extrakte vor allem in einem Druckbereich von 0,01 bis 50 mbar, vorzugsweise 0,05 bis 5 mbar, entsprechend einem Temperaturbereich von 110 bis 200°C.

Etwa 5 bis 25 Vol.-% des zugeführten Extrakts fallen als Kopfprodukt an, welches in der Regel alle übelriechenden Nebenbestandteile enthält.

Den Kolonnensumpf kann man mit den üblichen technischen Mitteln, beispielsweise mit einem Dünnfilmverdampfer, erhitzen.

Das so gereinigte α-Bisabolol kann ohne weitere Behandlung als Fixateur für Riechstoffe oder in Kosmetika eingesetzt werden.

Nach dem erfindungsgemäßen Verfahren kann man alle Stereoisomeren des α-Bisabolols, isoliert oder als Gemisch, reinigen.

Das Verfahren zeichnet sich dadurch aus, daß es einfach durchführbar und umweltschonend ist und hohe Ausbeuten an Wertprodukt liefert.

### Beispiel

In eine mit einer Metallpackung (CY-Packung der Fa. Sulzer AG) gefüllte Füllkörperkolonne von 1,70 m Höhe und 0,15 m Durchmesser mit 18 theoretischen Böden, an deren Kopf ein Druck von 0,3 bis 0,5 mbar herrschte, wurden auf Höhe des 12. Bodens (von unten gezählt) bei 128 bis 132°C stündlich 8 kg Candeia-Öl gegeben, welches 80 Gew.-% (-)-α-Bisabolol enthielt.

Die Kolonne wurde mit Hilfe eines Wärmeträgeröls beheizt. Zusätzlich dazu wurde die Sumpfphase über einen Dünnfilmverdampfer erhitzt.

Bei einem Rücklaufverhältnis von 6:1 fielen am Kolonnenkopf (85°C) stündlich 1,5 kg Destillat, im Kolonnensumpf (240°C) 0,5 kg Sumpfprodukt und auf Höhe des 6. Bodens bei 130 bis 138°C 6 kg dampfförmiges (-)-α-Bisabolol an.

Die Destillationsausbeute an (-)-α-Bisabolol betrug somit 94 %, bezogen auf dessen Gehalt im eingesetzten Candeia-Öl.

Das so gewonnene (-)-α-Bisabolol hatte eine Reinheit von 97 % und ließ sich in dieser Form unmittelbar zur Herstellung von kosmetischen Präparaten verwenden.

## Patentansprüche

1. Verfahren zur Reinigung von α-Bisabolol durch Destillation eines α-Bisabolol enthaltenden Pflanzenextraktes unter vermindertem Druck, dadurch gekennzeichnet, daß man das gereinigte α-Bisabolol in der unteren Hälfte einer Destillationskolonne als Seitenprodukt abzieht und den Extrakt oberhalb des Seitenabzugs zuführt.

2. Verfahren zur Reinigung von α-Bisabolol gemäß Anspruch 1, dadurch gekennzeichnet, daß zwischen Zulauf und Seitenabzug und zwischen Kolonnensumpf und Seitenabzug je 2 bis 20 theoretische Böden liegen.

## Claims

1. A process for purifying α-bisabolol by distillation of a plant extract containing α-bisabolol under reduced pressure, wherein the purified α-bisabolol is drawn off in the lower half of a distillation column as side product, and the extract is fed in above the side outlet.

2. A process for purifying α-bisabolol as claimed in claim 1, wherein in each case from 2 to 20 theoretical plates are located between inlet and side outlet and between column bottom and side outlet.

## Revendications

1. Procédé de purification d'α-bisabolol par distillation d'un extrait de plante contenant de l'α-bisabolol sous pression réduite, caractérisé en ce que l'α-bisabolol purifié est soutiré comme produit de soutirage latéral dans la moitié inférieure d'une colonne de distillation et en ce que l'extrait est introduit au-dessus du soutirage latéral.

2. Procédé de purification d'α-bisabolol selon la revendication 1, caractérisé en ce que les espaces compris entre l'alimentation et le soutirage latéral, et entre le fond de colonne et le soutirage latéral correspondent chaque fois à 2 à 20 plateaux théoriques.
